# EUROPEAN PATENT APPLICATION

(11) **EP 4 494 666 A1**
(43) Date of publication of application: **22.01.2025**
(21) Application number: 24189284.3
(22) Date of filing: 17.07.2024
(51) Int. Cl.: A61L 27/18, A61L 27/54, A61L 27/58

(54) **COMPOSITION FOR OBTAINING A TISSUE REGENERATION OR RECONSTRUCTION MATERIAL, FOR EXAMPLE BONE TISSUE, OR FOR DRUG DELIVERY AS WELL AS SYSTEM AND METHOD FOR OBTAINING SUCH COMPOSITION**

(30) Priority: 18.07.2023 IT 202300015093
(71) Applicant: Brenta S.r.l., 36045 Lonigo (VI) (IT)
(72) Inventor: CASTELLIN, Andrea, 36045 Lonigo (Vicenza) (IT); SPONCHIA, Gabriele, 36045 Lonigo (Vicenza) (IT)
(74) Representative: Feltrinelli, Secondo Andrea

(57) **Abstract**

The present invention relates to a composition for obtaining a tissue regeneration or reconstruction material, for example bone tissue, or a material capable of transporting and/or releasing at least one pharmaceutical, nutraceutical and/or cosmeceutical active ingredient.

## Description

### TECHNICAL FIELD OF THE INVENTION

The present invention concerns a composition for obtaining a tissue regeneration or reconstruction material, for example of bone tissue, or a material capable of transporting and/or releasing at least one active pharmaceutical, nutraceutical, cosmeceutical ingredient (drug delivery), whether or not integrated with the tissue, as well as a system and a process for producing such a composition.

### STATE OF THE PRIOR ART

The field of materials useful for tissue reconstruction, for example of bone tissue, is in continuous evolution to try to obtain new compounds for the integration of wounds and fractures at the bone level, which are able to allow regrowth, at the same time as a drug carrier capacity.

Furthermore, there is often a need to have a material available to be used as a *drug delivery* solution to transport and/or release active pharmaceutical, nutraceutical, cosmeceutical ingredients, even of the type not integrating with the tissue in question.

According to a solution not according to the invention, a material for tissue regeneration is a polyester based on citric acid and glycerol, or other diols.

However, this solution does not adapt satisfactorily to the desired shape, since, when applied, it spreads chaotically, "wetting" the respective substrate and takes many hours, even 24, before obtaining a solid mass.

### OBJECTS OF THE INVENTION

An object of the present invention is to provide a new composition for obtaining a tissue regeneration or reconstruction material, for example of bone tissue, or a material capable of transporting and/or releasing at least one active pharmaceutical, nutraceutical, cosmeceutical ingredient (*drug delivery*) whether or not integrated with the tissue as well as a new system for the obtainment of such a composition.

Another object of the present invention is to provide a composition as indicated above that adapts to the desired shape on which it is to be applied.

A further object of the present invention is to provide a composition as indicated above that is also water-soluble.

Not least, the object of the present invention is to provide a composition as indicated above that is biocompatible and biodegradable.

Another object of the present invention is to provide a composition as indicated above with properties of radiopacity and photoluminescence for diagnostic and theranostic purposes.

A further object of the present invention is to provide a composition as indicated above that is capable of carrying pharmacologically active active ingredients.

According to one aspect of the invention, a composition according to claim 1 is provided.

According to another aspect of the invention, a system according to claim 8 is provided.

According to another aspect of the invention, a process according to claim 19 is provided.

The dependent claims refer to preferred and advantageous embodiments of the invention.

### BRIEF DESCRIPTION OF THE DRAWINGS

Other features and advantages of the invention will be more evident from the description of an embodiment of a composition and system also thanks to the attached drawings in which:
- figure 1 is a block diagram illustrating a process of obtaining a composition according to the present invention;
- figure 2 illustrates the behavior of a composition according to the present invention when applied on a support; and
- figure 3 illustrates the behavior of a composition not according to the present invention applied on a support similar to that of figure 2.

### EMBODIOMENTS OF THE INVENTION

The present invention concerns a composition for obtaining a tissue regeneration or reconstruction material, for example of bone tissue, or a material capable of transporting and/or releasing at least one active pharmaceutical, nutraceutical, cosmeceutical ingredient (*drug delivery*), whether or not integrated with the tissue comprising at least one first element containing at least two at least two carboxyl endings (-COOH).

In this description, the term "element" does not refer exclusively to a chemical element, but can also refer to a compound formed by multiple elements joined together.

The presence of at least two carboxylic acid endings is necessary for the formation of the polymer network that is desired.

The first element could for example be chosen from the group consisting of citric acid, aconitic acid, malic acid, maleic acid, malonic acid, adipic acid, succinic acid, suberic acid or mixtures thereof.

Preferably, the first element comprises or consists of citric acid, due to its compatibility for the intended use, its low hazard and the ability to stabilize a metal center (e.g. zirconium).

The composition further comprises at least one second element containing at least two alcoholic endings (-OH), for example a diol, a triol, a polyphenol, etc., optionally glycerol, 1,8-octanediol, 1,12-dodecanediol, tannic acid, propylene glycol, one or more sugar alcohols (for example erythritol, xylitol, sorbitol, mannitol), gluconic acid or mixtures thereof.

With particular reference to the choice of glycerol, it is preferable for its compatibility with the intended use and the absence of danger when using it. Similar considerations apply to 1,8-octanediol and 1,12-dodecanediol.

The presence of at least two alcohol endings is necessary for the formation of the network.

The composition also includes zirconium. Zirconium is preferable for its compatibility with the intended use and its compatibility.

If desired, the composition also includes an additional element, chosen from the group consisting of calcium, copper and other metals that can be coordinated by the first element (for example, citric acid) or mixtures thereof.

Relative to the term "coordinable", as it is known, coordinability (which applies to zirconium and to the possible additional element) is the ability of a molecule containing more than one functional group (i.e. particular constructions of atoms), even different ones, suitable for binding other external molecules (for example metal ions) with which they have chemical affinity or on which they induce a physical stabilization (for example electric charge). Thus, for example, citric acid has three functional groups, which are able to effectively stabilize metal cations, thanks to electrical attraction.

In this description, an explicit description has been provided only for a composition and system with citric acid as the first element, glycerol as the second element and zirconium, but it should be understood that all combinations of all the preferred first and second elements (together with zirconium and possibly the additional element) specified above are to be considered as included in the content of this description.

The composition could clearly include other components in addition to the first element (if desired citric acid), zirconium and second element.

The composition is in the form of a gel.

According to a further version, especially in the case in which the composition is used as a drug delivery, the composition can be in the form of a film or membrane, as well as of gel.

If desired, the composition consists of a polymer or better of a polyester.

The starting solution or system from which the gel/polymer or film or membrane is obtained by evaporation (as will be better explained later) of solvents (the latter constituting 50-80%, for example 60-70% of the starting solution), is a final composition or dry mass containing 20-50%, for example 30-40% of the initial mass. This final composition or dry mass will preferably consist of 5-20%, for example 10-15% by weight of zirconium, the remaining 80-95%, if desired 85-90% being instead formed by the first element (for example citric acid) and the second element (such as glycerol) in the form of partially or completely polymerized polyester.

The present invention also relates to a system comprising or consisting of three components for obtaining, when mixed together, a composition as indicated above.

More specifically, the system comprises:
- a first component including or consisting of one or more first elements, for example citric acid, preferably solubilized with a solvent, for example a solvent selected from the group consisting of ethanol, optionally absolute ethanol, methanol, propanol, isopropanol, longer chain alcohols or mixtures thereof,
- a second component including or consisting of at least one second element, for example a diol, optionally mixed until homogeneous (i.e. obtaining a single clear phase without the presence of a phase separation meniscus) with a solvent, for example a solvent selected from the group consisting of ethanol, optionally absolute ethanol, methanol, propanol, isopropanol, longer chain alcohols or mixtures thereof, and
- a third component including or consisting of zirconium, optionally zirconium propoxide, preferably mixed with a solvent, for example a solvent selected from the group consisting of ethanol, optionally absolute ethanol, methanol, propanol, isopropanol, longer chain alcohols or mixtures thereof.

If desired, the third component also comprises an additional element mixed with zirconium, which additional element is chosen from the group consisting of calcium, copper and other metals that can be coordinated by the first element (for example, citric acid) or mixtures thereof.

Advantageously, in the system, the molar ratio between the first (citric acid if desired) and the second (diol if desired) element is between 1 and 3, even more advantageously between 1.8 and 2.2, if desired approximately 2.

Preferably, the molar ratio between zirconium and the first (citric acid if desired) element is between 0.1 and 0.5, even more preferably between 0.35 and 0.4, if desired approximately 3/8.

The production system may clearly include or not other components in addition to the first, second and zirconium, but in any case the latter components constitute at least 70% or 80% or 90% by weight of the system.

It is also reiterated that the starting solution or system from which the gel/polymer or film or membrane is obtained by evaporation (as will be better explained later) of solvents (the latter constituting 50-80%, for example 60-70% of the starting solution), is a final dry composition or mass containing 20-50%, for example 30-40% of the initial mass. This final dry composition or mass will preferably be made up of 5-20%, for example 10-15% of zirconium, the remaining 80-95%, if desired 85-90% being instead formed by the first element (for example citric acid) and the second element (such as glycerol) in the form of partially or completely polymerized polyester.

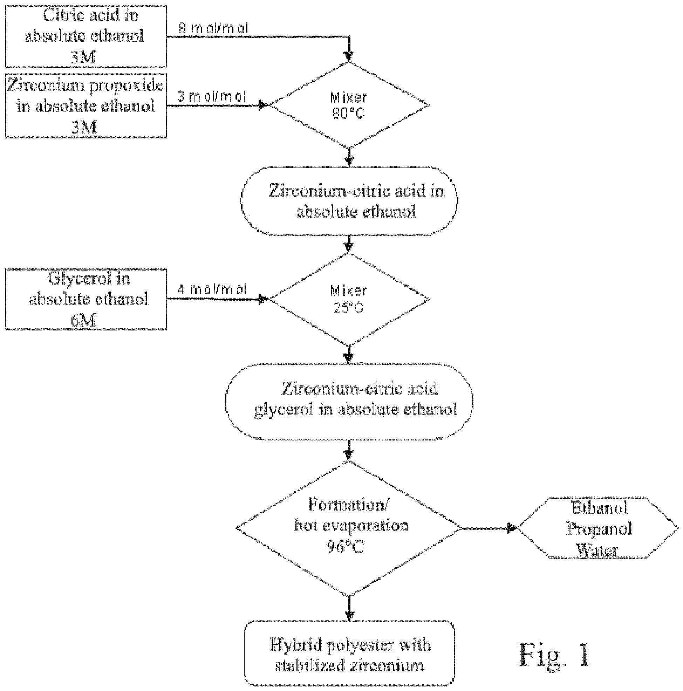

A composition according to the present invention can be obtained by any suitable process (see figure 1), for example by mixing (the mixing can be obtained by any suitable means that allows the formation of a homogeneous mixture formed by a single phase) first the first component and the third component, then following the formation of the third-first component solution or better still element, for example zirconium-citric acid, preferably by heating, the second component is introduced and then one heats so as to obtain a polymer or better still a polyester, if desired as a homogeneous and/or transparent solid gel.

If desired, in a process according to the present invention, a solution of the first component, such as citric acid in a solvent, such as ethanol or one of the others indicated above, is first mixed with the organic precursor of the third component, including or consisting of zirconium, for example zirconium propoxide (Zr(OPr)4 or ZP), zirconium butoxide or other organic precursors of the same metal in the form of alkoxides, such as zirconium butoxide, obtaining a stable and homogeneous solution following gentle heating, for example in a microwave or by other means at a temperature between 40°C and 100°C, for example approximately 70-90°C, preferably 80°C, after placing the components in a closed container, for the dissolution of the precipitate obtained at the time of mixing between ZP and citric acid.

From a stoichiometric point of view, the first element, such as citric acid, is preferably in excess with respect to zirconium.

Given the nature of the components and the relative stability of the solution obtained in the presence of water, it was possible to deduce that zirconium is stabilized in the presence of the first element, such as citric acid. In fact, it is known from the literature that the organic precursors of zirconium, including zirconium propoxide, are unstable in water, leading to the precipitation of zirconium oxide.

Following the formation of the zirconium-citric acid solution, the second component, i.e. the diol, for example glycerol, useful for polymerization is introduced.

The first (such as citric acid) and second (such as glycerol) elements are able, upon heating, to generate a polymer, thanks to the esterification reaction that occurs between the carboxylic groups of the first element and the alcoholic groups of the second element. The reaction produces as waste, for example, a water molecule for each ester bond formed. The polymer obtained according to the present invention can therefore be classified as a polyester.

This polyester is, in accordance with a solution not according to the present invention, obtained with an equimolar stoichiometric ratio of the two components (if desired citric acid and glycerol) (Wrzecionek et al., 2021; Zahlan et al., 2019).

According to the present invention, instead, the second element (glycerol if desired) introduced into the first element-zirconium solution (citric acid-zirconium if desired) is advantageously lower than the equimolar value, since part of the carboxylic groups of the first element present are engaged in the stabilization of the metal center, namely the zirconium. This particular stoichiometry allows to obtain a polymeric network (polyester) that at the same time keeps the zirconium stably inside it.

Once the complete first-third-second element solution has been obtained, for example citric acid-zirconium-glycerol, polymerization of the solution occurs or, preferably, is induced by heating (preferably both after mixing the first and third components, and after introducing the second component), so that the evaporation of the solvents present (for example ethanol, propanol and reaction water) allows the polymer or better still the polyester to be obtained, for example as a homogeneous and transparent solid gel.

This gel is advantageously water-soluble following dispersion in water, with higher times the greater the degree of drying of the gel. In fact, water is responsible for the reverse hydrolysis process to esterification, causing the redissolution of the formed network.

In essence, according to the present invention, it is possible to exploit the complexing capacity of the first element (citric acid if desired) towards zirconium cations, to obtain a hybrid gel of for example citrate-glycerol-zirconium.

Zirconium also has the advantage that it is widely used in the medical field, also thanks to its radiopacity.

This hybrid gel, in addition to containing elements compatible with bone tissue (zirconium, phosphorus, calcium, copper, iron, zinc, cobalt, tantalum, yttrium, lithium, sodium, potassium, magnesium, strontium, gallium; lanthanides such as cerium and europium; metalloids such as boron, etc.) could be obtained with different shapes depending on the forming-drying technique, allowing a certain applicative versatility.

With reference to this aspect, depending on the hot evaporation technique of the solvents that allows the polymerization reaction to be obtained, thin coatings, films, controlled depositions according to 3D printing, molding or extrusion forming can be obtained.

Finally, thanks to the fact that the dry gel takes on a solid form that can be handled and used for the impregnation process, it is possible to impregnate the aforementioned gel with particular molecules, such as pharmaceutical API, nutraceuticals, cosmeceuticals active ingredients, or any molecule useful for an antibacterial and/or antiproliferative activity such as oncological chemotherapeutic products, for example through the technique known as Diffusion Supported Loading (DiSupLo), and this would allow the production of a multifunctional material for bone integration, allowing the delivery of drugs useful for antiproliferative, antibiotic or antibacterial action.

With regard to this aspect, through impregnation of the polymer network formed, the molecule of interest in solid form is trapped inside thanks to a migration process (exploiting physical principles such as capillarity) carried by solvent vapors that helps its dispersion in the polymer.

Subject-matter of the present invention is also a composition for use in the treatment of tissue regeneration or reconstruction, for example of bone tissue, or as a material capable of transporting and/or releasing at least one active pharmaceutical, nutraceutical, cosmeceutical ingredient (*drug delivery*), whether or not integrated with the tissue, or in any case for diagnostic and/or theranostic purposes, in the biomedical field, such as regenerative and reconstructive tissue engineering, also for cardiovascular, muscular, epidermal/skin or nervous system applications.

A specific embodiment of a process according to the present invention for obtaining a composition according to the present invention is reported below, in which there is a molar ratio of zirconium propoxide:citric acid:glycerol=3:8:4

2g of citric acid are solubilized with 3.6mL of absolute ethanol and 1.8mL of zirconium propoxide are mixed until homogeneous with 1.3mL of absolute ethanol.

The two solutions are mixed so as to obtain an opaque suspension that is solubilized by heating, that is, a few minutes in a microwave or 1h in an oven at 80°C in a closed container.

Once cooled, the thus obtained mixture or solution is stable and no reprecipitation occurs.

Subsequently, 0.5g of glycerol is mixed until homogeneous with 0.9mL of absolute ethanol with the aim of decreasing its viscosity.

The glycerol solution is then added to the citric acid-zirconium solution, and the resulting mixture or solution is homogeneous, clear and fluid.

The mixture or solution obtained is deposited on a substrate heated to 96°C, so as to obtain a gel with a defined shape that solidifies in a few minutes. The temperature allows the polymerization reaction to occur and at the same time to remove the solvents present (ethanol, propanol and reaction water).

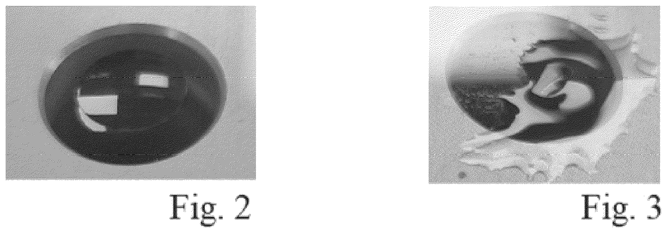

Figures 2 and 3 show the different behavior highlighted in hot forming on a glass substrate heated to 96°C, between a composition (figure 2) according to the present invention obtained as above indicated and a solution prepared with the same process described, but without zirconium (figure 3).

From the figures it emerges that for the same deposited volume (20µL) and deposition technique:
- a composition according to the present invention maintains a well-defined shape (convex lens), while a composition not according to the invention spreads chaotically, "wetting" the substrate;
- a composition according to the present invention passes from the liquid state to the solidified gel state within 1h from deposition, a composition not according to the invention after 8h from deposition still appears as a highly viscous liquid, while it is necessary to wait 24h to obtain a solid and elastic mass;
- a composition according to the present invention maintains a rigid and flexible consistency that becomes increasingly fragile as the drying time increases (formation of cracks on the transparent lens), while a composition not according to the invention instead has a consistency similar to rubber.

The two compositions are similar with regard to the transparent color and the ability to dissolve in water without generating precipitates.

As it will be understood thanks to the present invention it is possible to obtain a composition:
- new for obtaining a tissue regeneration or reconstruction material, for example of a bone tissue, or of a material capable of transporting and/or releasing at least one active pharmaceutical, nutraceutical, cosmeceutical ingredient (drug delivery), integrating or not with the tissue,
- able to adapt to the desired shape (molding process support),
- water-soluble, despite the presence of the transition metal and the organometallic complex,
- biocompatible and biodegradable,
- with radiopacity and photoluminescence properties for diagnostic and theranostic purposes,
- able to convey pharmacologically active active ingredients.

Modifications and variations of the invention are possible within the scope defined by the claims.

## Claims

1. Composition for obtaining a tissue regeneration or reconstruction material, for example bone tissue, or a material capable of transporting and/or releasing at least one pharmaceutical, nutraceutical and/or cosmeceutical active ingredient, comprising at least one first element containing at least two carboxyl endings (-COOH) and at least one second element containing at least two alcoholic endings (-OH), wherein said composition includes zirconium, said zirconium constituting among 5 and 20% by weight of said composition.

2. Composition according to claim 1, wherein said at least one first element is selected from the group consisting of citric acid, aconitic acid, malic acid, maleic acid, malonic acid, adipic acid, succinic acid, suberic acid or mixtures thereof.

3. Composition according to claim 1 or 2, wherein said at least one second element consists of a diol, a triol, a polyphenol or mixtures thereof.

4. Composition according to claim 3, wherein said at least one second element consists of glycerol, 1,8-octanediol, 1,12-dodecanediol, propylene glycol, at least one alditol, gluconic acid, tannic acid or mixtures thereof.

5. Composition according to any one of the preceding claims, wherein said at least one first element consists of citric acid and said at least one second element consists of glycerol.

6. Composition according to any one of the preceding claims, consisting of a polyester.

7. Composition according to any one of the preceding claims, impregnated with pharmaceutical, nutraceutical, cosmeceutical active ingredients, or with at least one molecule for carrying out an antibacterial and/or antiproliferative activity.

8. Composition according to any one of the preceding claims, wherein said zirconium constitutes among 10 and 15% by weight of the composition.

9. System comprising or consisting of three components for obtaining, when mixed together, a composition for obtaining a tissue regeneration or reconstruction material, for example bone tissue, or a material capable of transporting and/or releasing at least one pharmaceutical, nutraceutical and/or cosmeceutical active ingredient, wherein:
- a first component of said at least three components comprises or consists of at least one first element containing at least two carboxyl endings (-COOH),
- a second component of said at least three components comprises or consists of at least one second element containing at least two alcoholic endings (-OH),
- a third component of said at least three components comprises or consists of zirconium,
where the molar ratio between zirconium and the first element is between 0.1 and 0.5.

10. System according to claim 9, wherein said at least one first element is selected from the group consisting of citric acid, aconitic acid, malic acid, maleic acid, malonic acid, adipic acid, succinic acid, suberic acid or mixtures thereof.

11. System according to claim 9 or 10, wherein said at least one second element consists of a diol, a triol, a polyphenol, such as for example glycerol, 1,8-octanediol, 1,12-dodecanediol, propylene glycol, at least one alditol, gluconic acid, tannic acid or mixtures thereof.

12. System according to claim 9, 10 or 11, wherein said at least one first component comprises said at least one first element solubilized with a solvent selected from the group consisting of ethanol, methanol, propanol, isopropanol, longer chain alcohols or mixtures thereof.

13. System according to claim 9, 10, 11 or 12, wherein said zirconium comprises or consists of zirconium propoxide.

14. System according to claim 9 or 10 or 11 or 12 or 13, wherein said third component comprises zirconium mixed with a solvent selected from the group consisting of ethanol, methanol, propanol, isopropanol, longer chain alcohols or mixtures thereof.

15. System according to any one of claims 9 to 14, wherein said second component comprises said at least one second element mixed up to homogeneity with a solvent selected from the group consisting of ethanol, methanol, propanol, isopropanol, longer chain alcohols or their blends.

16. System according to any one of claims 9 to 15, wherein the molar ratio between zirconium and first element is between 0.35 and 0.4.

17. System according to any one of claims 9 to 16, wherein the molar ratio between the first and second element is between 1 and 3.

18. System according to any one of claims from 9 to 17, wherein the molar ratio between the first and the second element is between 1.8 and 2.2.

19. Process for obtaining a composition for obtaining a tissue regeneration or reconstruction material or a material capable of transporting and/or releasing at least one pharmaceutical, nutraceutical and/or cosmeceutical active ingredient, by means of a system according to any one of claims 9 to 18, wherein said first component and said third component are first mixed, after which, following the formation of the third-first component solution, the second component is introduced and then it is heated so as to obtain a polymer or a polyester.

20. Process according to claim 19, wherein a solution of said first element in a solvent is first mixed with said zirconium, thereby obtaining a stable and homogeneous solution after heating between 40°C and 100°C and then one proceeds to introduce the second component.

21. Composition for the use in the treatment of tissue regeneration or reconstruction, for example of bone tissue, or as a material capable of transporting and/or releasing at least one pharmaceutical, nutraceutical, cosmeceutical active ingredient, or in any case for diagnostic and/or theranostics purposes in the biomedical field, such as regenerative and reconstructive tissue engineering if desired for cardiovascular, muscular, epidermis/skin or nervous system applications, said composition being according to any one of claims 1 to 8 or being obtained with a process according to claim 19 or 20.
